# EUROPÄISCHE PATENTSCHRIFT

(11) **EP 3 119 262 B1**
(45) Veröffentlichungstag und Bekanntmachung des Hinweises auf die Patenterteilung: **15.07.2020**
(21) Anmeldenummer: 15709877.3
(22) Anmeldetag: 06.03.2015
(51) Int. Cl.: A61B 1/00, A61B 1/015, A61B 1/06, A61B 1/07

(54) **PUMPENVORRICHTUNG**
PUMP DEVICE
DISPOSITIF À POMPE

(30) Priorität: 18.03.2014 DE 102014204997
(43) Veröffentlichungstag der Anmeldung: 25.01.2017
(73) Patentinhaber: OLYMPUS Winter & Ibe GmbH, 22045 Hamburg (DE)
(72) Erfinder: WOLTER, Michael, 22307 Hamburg (DE)
(74) Vertreter: Glawe, Delfs, Moll
(86) Internationale Anmeldenummer: PCT/EP2015/054755
(87) Internationale Veröffentlichungsnummer: WO 2015/139972

(56) Entgegenhaltungen:
- WO-A1-2013/160443
- CN-U- 202 876 062
- US-A1- 2006 030 751

## Beschreibung

Die Erfindung betrifft Pumpenvorrichtungen zur Verwendung in Operationssälen sowie Anordnungen aus entsprechenden Pumpenvorrichtungen und Endoskopen.

Im Bereich der Medizintechnik für Operationssäle finden sich vielfach Pumpen als Teil von medizinischen Apparaten oder als gesonderte, mit medizinischen Apparaten verbundene Einheiten. Beispiele hierfür sind Absaugpumpen oder Herz-Lungen-Maschinen. Auch werden Pumpen zur Zuführung von Spülflüssigkeiten in ein Operationsgebiet verwendet, mit der reseziertes Gewebe und Blut aus dem Operationsgebiet ausgeschwemmt werden können.

Im Bereich der minimal-invasiven Chirurgie ist es bekannt, Spülflüssigkeit in das Operationsgebiet bzw. einen das Operationsgebiet umfassenden Körperhohlraum mithilfe einer Pumpe einzubringen. Der Körperhohlraum wird dabei durch die Spülflüssigkeit so gefüllt und geweitet, dass das Operationsgebiet für den Operateur durch ein Endoskop gut sichtbar ist. Die Spülflüssigkeit wird dabei regelmäßig durch das Endoskop selbst zu- und ggf. auch wieder abgeführt. Durch einen andauernden Spülflüssigkeitsstrom kann weiterhin eine Eintrübung des Sichtfeldes durch Schwebestoffe o. ä. vermieden werden. Außerdem kann reseziertes Gewebe und Blut ausgeschwemmt werden. Ein solches Endoskopsystem ist beispielsweise aus der US 2006/0030751 A1 bekannt. Die Präambel des Anspruchs 1 geht von diesem Dokument aus. Entsprechende, in Operationssälen verwendete Pumpen gemäß dem Stand der Technik liefern über ein an ihrem Gehäuse angeordnetes Display Informationen über ihren Betriebszustand, wie bspw. den grundsätzlichen Anschaltzustand der Pumpe oder die Durchflussmenge. Das Gehäuse der Pumpe bzw. das daran angeordnete Display befindet sich im Regelfall nicht im Blickfeld des Operateurs, so dass er sich während der Operation entsprechend zur Pumpe hindrehen oder sich bei ihm bei der Operation assistierende Personen nach dem Betriebszustand der Pumpe erkundigen muss, um die im Display angezeigten Informationen zu erhalten. Dieser Stand der Technik ist nachteilig, da in beiden genannten Fällen der Operateur in seiner Konzentration auf die eigentliche Operation gestört wird.

Der Erfindung liegt daher die Aufgabe zugrunde, eine Pumpe zu schaffen, welche die Nachteile aus dem Stand der Technik nicht mehr oder wenigstens nur in vermindertem Umfang aufweist.

Gelöst wird diese Aufgabe durch eine Pumpenvorrichtung gemäß dem Hauptanspruch sowie einer Anordnung gemäß Anspruch 11. Vorteilhafte Weiterbildungen sind Gegenstand der abhängigen Ansprüche.

Demnach betrifft die Erfindung eine Pumpenvorrichtung zur Verwendung in Operationssälen, umfassend eine Pumpeneinheit mit einer Pumpe und einer Steuerungseinheit, und einen an die Pumpeneinheit angeschlossenen Pumpenschlauch, wobei die Pumpeneinheit eine durch die Steuerungseinheit zur Anzeige des Betriebszustandes der Pumpeneinheit ansteuerbare Lichtquelle umfasst und der Pumpenschlauch eine Lichtleitfaser aufweist, die sich entlang des Pumpenschlauchs erstreckt, wobei das erste Ende der Lichtleitfaser zur Einkopplung von Licht der ansteuerbaren Lichtquelle angeordnet ist.

Die Erfindung betrifft weiterhin eine Anordnung aus einer erfindungsgemäßen Pumpenvorrichtung und einem Endoskop, welches zur Zuführung von Spülflüssigkeit in das Operationsgebiet ausgebildet ist, wobei das freie Ende des Pumpenschlauchs der erfindungsgemäßen Pumpenvorrichtung zur Zuführung von Spülflüssigkeit mit dem Endoskop verbunden ist.

Indem erfindungsgemäß eine sich entlang des Pumpenschlauchs erstreckende Lichtleitfaser vorgesehen ist, in die Licht von einer, den Betriebszustand der Pumpeneinheit widerspiegelnden Lichtquelle eingekoppelt wird, können die Informationen über den Betriebszustand der Pumpeneinheit in unmittelbarer Nähe zum Operationsgebiet zur Anzeige gebracht werden, so dass ein Operateur die entsprechenden Informationen praktisch beiläufig wahrnehmen kann, ohne dass er dabei in seiner Konzentration auf die eigentliche Operation gestört wird. Die Erfindung macht sich dabei den Umstand zu Nutze, dass das freie Ende des Pumpenschlauchs einer Pumpenvorrichtung zur Verwendung in Operationssälen regelmäßig in das oder nah an das eigentliche Operationsgebiet herangeführt wird, womit auch die erfindungsgemäß vorgesehene, sich entlang des Pumpenschlauchs erstreckende Lichtleitfaser in unmittelbarer Nähe zum Operationsgebiet geführt wird. Am zweiten Ende der Lichtleitfaser tritt das am ersten Ende eingekoppelte Licht wieder aus und ist für den Operateur als Lichtpunkt wahrnehmbar. Dieser Lichtpunkt liefert dem Operateur aufgrund der erfindungsgemäßen Ansteuerung der Lichtquelle Informationen über den Betriebszustand der Pumpe.

Das zweite Ende der Lichtleitfaser kann am Ende des Pumpenschlauchs angeordnet sein. Das zweite Ende der Lichtleitfaser ist in diesem Fall vorzugsweise in einem Bereich zwischen 0 cm und 5 cm vom freien Ende des Pumpenschlauchs entfernt angeordnet.

Wenn die Lichtleitfaser bis zum Ende des Pumpenschlauchs geführt ist, kann das Licht beispielsweise direkt in einen üblicherweise transparent ausgeführten Luer-Lock-Anschluss eingestrahlt werden, von welchem es dann diffus abgestrahlt wird. Dabei ist es möglich, dass die Lichtquelle nicht sichtbares Licht, beispielsweise UV-Licht, abgibt, welches dann durch ein z.B. am Schlauchanschluss angeordnetes Umwandlungselement, beispielsweise ein Phosphorelement, in sichtbares Licht umgewandelt und abgestrahlt wird.

Es kann auch bevorzugt sein, wenn das zweite Ende der Lichtleitfaser von dem freien Ende des Pumpenschlauchs zurückversetzt angeordnet ist. Durch eine entsprechende zurückversetzte Anordnung des zweiten Endes der Lichtleitfaser kann gewährleistet werden, dass das zweite Ende der Lichtleitfaser zwar im generellen Blickfeld des Operateurs angeordnet ist, gleichzeitig jedoch nicht das eigentliche Operationsgebiet ausleuchtet, selbst wenn das freie Ende des Pumpenschlauchs in das Operationsgebiet geführt ist, bspw. zum Absaugen von Flüssigkeiten. Das zweite Ende der Lichtleitfaser kann vorzugsweise 10 cm bis 50 cm, weiter vorzugsweise 20 cm bis 40 cm vom freien Ende des Pumpenschlauchs angeordnet sein.

Es ist bevorzugt, wenn die Lichtleitfaser zwischen ihren beiden Enden eine oder mehrere Störstellen zum Lichtaustritt aufweist. Jede Störstelle ist für den Operateur dann als Lichtpunkt wahrnehmbar. Insbesondere wenn mehr als eine Störstelle vorgesehen ist, kann so gewährleistet werden, dass unabhängig von der Führung des Pumpenschlauchs in oder an das Operationsgebiet wenigstens eine Störstelle und damit wenigstens ein Lichtpunkt im generellen Blickfeld des Operateurs liegt.

Die Störstellen sind vorzugsweise als Kerben auf der Außenseite der Lichtleitfaser ausgebildet. Sind mehrere Störstellen vorgesehen, ist bevorzugt, wenn die Abstände zwischen zwei jeweils benachbarten Störstellen ausgehend vom ersten Ende der Lichtleitfaser entlang des Pumpenschlauchs zum zweiten Ende der Lichtleitfaser hin abnehmen. So kann bei im Übrigen geeigneter Wahl der Abstände erreicht werden, dass pro Längenabschnitt des Schlauchs ungefähr die gleiche Lichtmenge abgegeben wird. Das zweite Ende der Lichtleitfaser kann bei dem Vorhandensein von Störstellen frei sein, und so als zusätzlicher Lichtpunkt dienen. Alternativ kann das zweite Ende der Lichtleitfaser aber auch abgedeckt sein, sodass ausschließlich die Störstellen als Lichtpunkte dienen.

Die ansteuerbare Lichtquelle ist vorzugsweise farbveränderlich. Dabei ist besonders bevorzugt, wenn die Farbveränderung der Lichtquelle durch die Steuerungseinheit gesteuert werden kann. Dies kann durch bewegliche Farbfilter erreicht werden. Es ist jedoch besonders bevorzugt, wenn die Lichtquelle mehrere unterschiedlich farbige Leuchtmittel umfasst, die einzeln hinsichtlich ihrer Intensität angesteuert werden können, sodass sich im Ergebnis der gewünschte Farbton der Lichtquelle einstellt. Vorzugsweise umfassen die unterschiedlich farbigen Leuchtmittel die Farben Rot, Grün und Blau, ggf. auch Weiß.

Die ansteuerbare Lichtquelle umfasst vorzugsweise Licht emittierende Dioden (LEDs). LEDs zeichnen sich durch ihre lange Lebensdauer auch bei vielen Ein- und Ausschaltvorgängen aus. LEDs sind darüber hinaus auch in den für die Einstellbarkeit über verschiedenfarbige Leuchtmittel bevorzugten Farben Rot, Grün, Blau und Weiß verfügbar.

Die Steuerungseinrichtung ist bevorzugt dazu ausgebildet, die Lichtquelle in Abhängigkeit der an der Pumpe eingestellten Durchflussrate pulsieren oder blinken zu lassen. "Pulsieren" bedeutet in diesem Zusammenhang, dass die Intensität der Lichtquelle rhythmisch schwankt, vorzugsweise jedoch ohne zu erlöschen. "Blinken" bedeutet ein rhythmisches An- und Abschalten der Lichtquelle.

Die Steuerungseinrichtung ist weiterhin bevorzugt dazu ausgebildet, die Farbe der Lichtquelle in Abhängigkeit von der Durchflussrichtung einzustellen. Dadurch kann sichergestellt werden, dass bspw. an den Störstellen und/oder dem zweiten Ende der Lichtleitfaser wahrnehmbare Leuchtpunkte an Pumpenschläuchen für den Zulauf andersfarbig leuchten als diejenigen an Pumpenschläuchen für den Ablauf.

Die Pumpeneinrichtung kann vorzugsweise auch einen oder mehrere Sensoren zur Überwachung von Betriebsparametern der Pumpeneinheit und/oder von Eigenschaften des zu pumpenden Fluids, wie bspw. dessen Temperatur, aufweisen. Die Steuerungseinrichtung ist dann bevorzugt zur Anzeige der Messwerte des oder der Sensoren durch die Lichtquelle ausgebildet, wobei vorzugsweise die Farbe der Lichtquelle in Abhängigkeit der Messwerte geändert wird. So kann bspw. die Temperatur des zu pumpenden Fluids gemessen werden und - sofern die Temperatur in einer vorgegebenen Temperaturspanne liegt - Grün als Farbe der Lichtquelle eingestellt werden, während bei Abweichungen der Temperatur von der vorgegebenen Spanne die Farbe der Lichtquelle auf Rot geändert wird. Es ist aber auch möglich, dass Messwerte oder Abweichungen der Messwerte von Sollwerten durch schnelles Blinken des Leuchtmittels o. ä. angezeigt wird.

Die Lichtleitfaser kann auf der Außenseite des Pumpenschlauchs angeordnet sein. Die Lichtleitfaser kann dabei bspw. durch Kleben an dem Pumpenschlauch befestigt sein. Es ist auch möglich, dass die Lichtleitfaser mit dem Pumpenschlauch coextrudiert ist. Ist der Pumpenschlauch wenigstens teilweise transparent, kann die Lichtleitfaser auch in den Pumpenschlauch eingebettet sein.

Der Pumpenschlauch kann auch ein, vorzugsweise wenigstens teilweise transparenter, Multilumenschlauch sein, wobei die Lichtleitfaser durch eine Kammer des Multilumenschlauchs geführt oder mit dem Multilumenschlauch coextrudiert ist. In den übrigen Kammern des Multilumenschlauchs können neben einer Kammer für den Flüssigkeitstransport bspw. Lichtleitfasern für Endoskopiebeleuchtung oder Laserapplikationen etc. geführt sein.

Bei der Pumpenvorrichtung handelt es sich bevorzugt um eine Pumpenvorrichtung für die Spülflüssigkeit bei minimalinvasiven Eingriffen über ein Endoskop, wobei die Spülflüssigkeit durch das Endoskop selbst in das Operationsgebiet eingebracht wird. Das freie Ende des Pumpenschlauchs ist hierfür bevorzugt für den Anschluss an ein entsprechendes Endoskop ausgebildet. Die Erfindung betrifft auch eine entsprechende Anordnung aus einer erfindungsgemäßen Pumpeneinheit und einem solchen Endoskop, wobei das freie Ende des Pumpenschlauchs mit dem Endoskop verbunden ist. Zur Erläuterung wird auf die vorstehenden Ausführungen verwiesen.

Es ist auch möglich, dass die ansteuerbare Lichtquelle nicht an der Pumpeneinheit sondern vielmehr am Pumpenschlauch angeordnet ist und über eine elektrische Leitung, die sich anstelle der Lichtleitfaser entlang des Pumpenschlauchs erstreckt, mit der Steuerungseinheit der Pumpeneinheit verbunden ist. Die Position der Lichtquelle an dem Pumpenschlauch kann dabei beliebig gewählt werden, insbesondere kann sie aber am freien Ende des Pumpenschlauchs angeordnet sein.

Die Erfindung wird nun anhand vorteilhafter Ausführungsformen unter Bezugnahme auf die beigefügten Zeichnungen beispielhaft beschrieben. Es zeigen:
- Figur 1:: ein erstes Ausführungsbeispiel einer erfindungsgemäßen Pumpenvorrichtung;
- Figur 2:: ein zweites Ausführungsbeispiel einer erfindungsgemäßen Pumpenvorrichtung;
- Figur 3a-b:: zwei beispielhafte Ausführungsvarianten von Pumpenschläuchen für die Pumpenvorrichtungen gemäß Figuren 1 und 2; und
- Figur 4: ein ersten Ausführungsbeispiel einer erfindungsgemäßen Anordnung aus Pumpenvorrichtung und Endoskop.

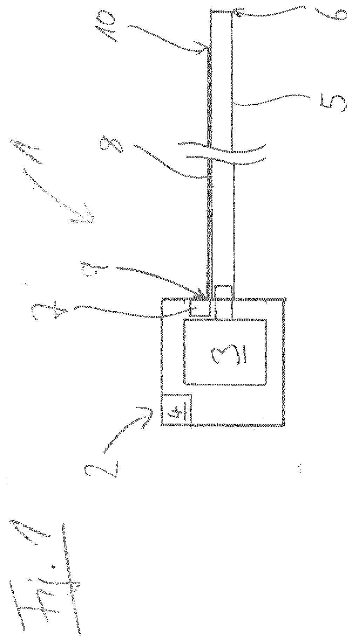

In Figur 1 ist eine erfindungsgemäße Pumpenvorrichtung 1 zur Verwendung in Operationssälen schematisch dargestellt. Die Pumpenvorrichtung 1 umfasst eine Pumpeneinheit 2 mit einer Pumpe 3 und einer Steuerungseinheit 4. Über die Steuerungseinheit 4 kann die Pumpe 3 gesteuert werden. An der Pumpeneinheit 2 können hierfür noch Bedienelemente (nicht dargestellt) vorhanden sein, über die die Pumpeneinheit 2 betätigt werden kann. Bspw. kann über ein entsprechendes Bedienelement die gewünschte Durchflussrate der Pumpe 3 eingestellt werden. Aus Gründen der Übersichtlichkeit sind die Bedienelemente und jedwede Steuerungsleitungen nicht dargestellt.

Mit der Pumpeneinheit 2 ist ein Pumpenschlauch 5 verbunden, so dass durch die Pumpe 3 der Pumpeneinheit 2 Fluide durch den Pumpenschlauch 5 gefördert werden können. Dabei kann die Pumpe 3 beispielsweise Spülflüssigkeit aus einem nicht dargestellten Reservoir durch den Pumpenschlauch 5 fördern, so dass die Spülflüssigkeit am freien Ende 6 des Pumpenschlauchs 5 austritt. Es ist auch möglich, dass die Pumpe 3 der Pumpeneinheit 2 in die entgegengesetzte Richtung arbeitet, so dass ein Fluid am freien Ende 6 des Pumpenschlauchs 5 angesaugt und durch den Pumpenschlauch 5 in einen nicht dargestellten Auffangbehälter gefördert wird.

An der Pumpeneinheit 2 ist weiterhin eine Lichtquelle 7 vorgesehen. Die Lichtquelle 7 ist hinsichtlich ihres Einschaltzustandes sowie ihrer Intensität von der Steuerungseinheit 4 ansteuerbar.

Die Lichtquelle 7 kann ein einzelnes Leuchtmittel, bspw. eine Licht emittierende Diode (LED) umfassen. Alternativ ist es auch möglich, dass die Lichtquelle 7 mehrere verschiedenfarbige, einzeln ansteuerbare LEDs - insbesondere in Rot, Grün, Blau und Weiß - aufweist, so dass das von der Lichtquelle 7 insgesamt abgegebene Licht zusätzlich in seiner Farbe durch Veränderung der Intensität der einzelnen verschiedenfarbigen Leuchtmittel eingestellt werden kann. In diesem Fall ist die Steuerungseinheit 4 dazu ausgebildet, die Farbe bzw. Farbveränderungen der Lichtquelle 7 zu steuern.

Entlang des Pumpenschlauchs 5 ist eine Lichtleitfaser 8 vorgesehen. Die Lichtleitfaser 8 ist mit ihrem ersten Ende 9 so angeordnet, dass das Licht der Lichtquelle 7 in die Lichtleitfaser 8 eingekoppelt wird. Das am ersten Ende 9 in die Lichtleitfaser 8 eingekoppelte Licht tritt am zweiten Ende 10 der Lichtleitfaser 8 wieder aus und ist dort als Lichtpunkt wahrnehmbar. Das zweite Ende 10 der Lichtleitfaser 8 ist dabei am freien Ende 6 des Pumpenschlauchs 5, genauer 5 cm von dem freien Ende 6 des Pumpenschlauchs 5 entfernt angeordnet.

Die Steuerungseinheit 4 ist dazu ausgebildet, die Lichtquelle 7 in Abhängigkeit vom Betriebszustand der Pumpeneinheit 2 und insbesondere der Pumpe 3 zu steuern. Insbesondere kann die Lichtquelle 7 parallel zum An- und Abschaltzustand der Pumpe 3 an- und abgeschaltet werden. Weiterhin kann die Steuerungseinheit 4 die Lichtquelle 7 derart steuern, dass die Lichtquelle 7 in Abhängigkeit der an der Pumpe 3 eingestellten Durchflussrate pulsiert oder blinkt. Darüber hinaus ist es möglich, dass im Falle einer farbver-änderlichen Lichtquelle 7 die Durchflussrichtung des Fluids durch den Pumpenschlauchs 5, die von der Pumprichtung der Pumpe 3 bestimmt wird, zu verändern.

Da das freie Ende 6 des Pumpenschlauchs 5 bei Pumpvorrichtungen 1 für Operationssäle, wie sie in Figur 1 dargestellt ist, regelmäßig in das oder zumindest nahe an das eigentliche Operationsgebiet herangeführt wird, und das zweite Ende 10 der Lichtleitfaser 8 an diesem freien Ende 6 des Pumpenschlauchs 5 angeordnet ist, erhält der Operateur die entsprechenden Informationen in Form eines Lichtpunkts am zweiten Ende 10 der Lichtleitfaser 8 in seinem Blickfeld. Der Lichtpunkt gibt nämlich aufgrund der entsprechenden Einkopplung von der Lichtquelle 7 Informationen über den Betriebszustand der Pumpeneinheit 2 wieder. Insbesondere ist es - anders als im Stand der Technik - nicht mehr erforderlich, dass der Operateur zur Abfrage des Betriebsstatus der Pumpeneinheit 2 seinen Blick von dem Operationsgebiet ab, hin zu einem Display an einer Pumpe wenden oder eine ihm assistierende Person über den Betriebszustand einer Pumpe befragen muss.

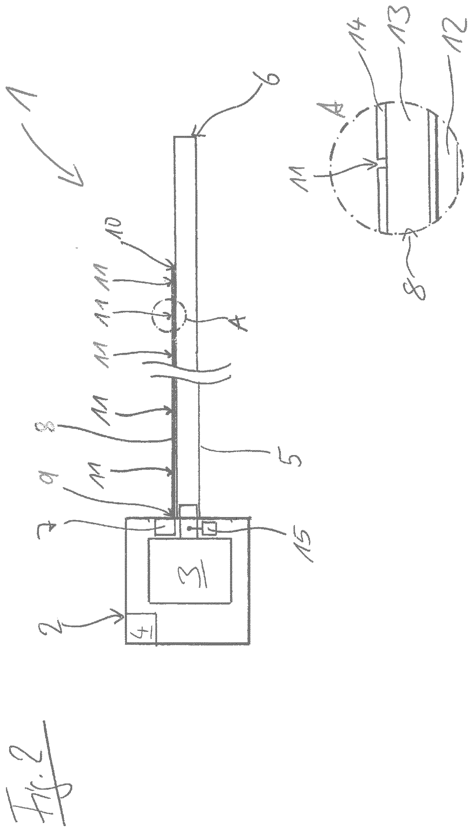

In Figur 2 ist ein zweites Ausführungsbeispiel einer erfindungsgemäßen Pumpenvorrichtung 1 dargestellt, welches in weiten Teilen demjenigen aus Figur 1 entspricht. Im Folgenden wird daher lediglich auf die Unterschiede des Ausführungsbeispiels gemäß Figur 2 gegenüber demjenigen aus Figur 1 eingegangen. Im Übrigen wird auf die obigen Ausführungen verwiesen

Bei dem Ausführungsbeispiel gemäß Figur 2 ist die Lichtleitfaser 8 nicht bis zum freien Ende 6 des Pumpenschlauchs 5 geführt. Vielmehr ist das zweite Ende 10 der Lichtleitfaser gegenüber dem freien Ende 6 des Pumpenschlauchs 5 um 30 cm zurückversetzt angeordnet.

Darüber hinaus weist die Lichtleitfaser 8 zwischen ihrem ersten Ende 9 und ihrem zweiten Ende 10 eine Mehrzahl von Störstellen 11 auf, wobei die Störstellen 11 zum Lichtaustritt ausgebildet sind. An den Störstellen 11 tritt also ein Teil des am ersten Ende 9 eingekoppelten Lichts aus, womit jede Störstelle 11 für den Operateur als einzelner Lichtpunkt wahrnehmbar ist. In der Folge ist für den Operateur entlang des Pumpenschlauchs 5 eine Vielzahl von Lichtpunkten wahrnehmbar, die jeweils den Betriebszustand der Pumpeneinheit 2 anzeigen.

In der Detaildarstellung A in Figur 2 sind die Störstellen 11 näher gezeigt. Die auf der Außenseite der Wand 12 des Pumpenschlauchs 5 angeordnete Lichtleitfaser 8 umfasst einen lichtführenden Kern 13, der von einem Mantel 14 umgeben ist. Der Mantel 14 weist einen niedrigeren Brechungsindex als der lichtführende Kern 13 auf, so dass es grundsätzlich zu einer Totalreflexion am Übergang vom Kern 13 zum Mantel 14 kommt. Im Bereich der Störstellen 11 ist die Lichtleitfaser 8 eingekerbt, so dass der Mantel 14 unterbrochen ist. An den Einkerbungen kommt es in der Folge zu keiner Totalreflexion mehr. Vielmehr kann im Bereich der Störstelle 11 Licht aus dem lichtleitenden Kern 13 austreten. Dieser Lichtaustritt kann vom Operateur als Lichtpunkt an der Störstelle 11 wahrgenommen werden.

Der Abstand zwischen zwei jeweils benachbarten Störstellen 11 nimmt ausgehend von dem ersten Ende 9 der Lichtleitfaser zum zweiten Ende 10 der Lichtleitfaser ab. Dabei ist der Abstand zwischen den ausgehend von der Pumpeneinheit 2 nächstgelegenen beiden Störstellen 11 am größten, der Abstand zwischen den beiden, dem zweiten Ende 10 der Lichtleitfaser 8 nächstliegenden Störstellen 11 am kleinsten. Durch diese Maßnahme ist es möglich, dass pro Längenabschnitt des Schlauchs 5 ungefähr die gleiche Lichtmenge abgegeben wird. Der Fachmann ist dabei ohne weiteres in der Lage, die erforderlichen einzelnen Abstände zwischen zwei benachbarten Störstellen 11 geeignet zu wählen, um eine im Wesentliche gleiche Intensität an allen Störstellen 11 zu erhalten. Das zweite Ende 10 der Lichtleitfaser 3 ist beim Ausführungsbeispiel gemäß Figur 2 frei und ist somit ebenfalls als Lichtpunkt für den Operateur wahrnehmbar.

Die Pumpeneinheit 2 im Ausführungsbeispiel gemäß Figur 2 weist weiterhin einen Temperaturfühler 15 auf, mit dem die Temperatur des durch den Pumpenschlauch 5 geförderten Fluids gemessen wird. Die Steuerungseinheit 4 ist so ausgebildet, dass die Farbe der Lichtquelle 7 gemäß den Messwerten des Temperaturfühlers 15 verändert wird. Beispielsweise kann die Farbe der Lichtquelle 7 Grün sein, wenn sich die gemessene Temperatur in einem vorgegebenen Temperaturbereich bewegt. Liegt die gemessene Temperatur außerhalb dieses Temperaturbereich kann die Farbe der Lichtquelle 7 nach Rot verändert werden.

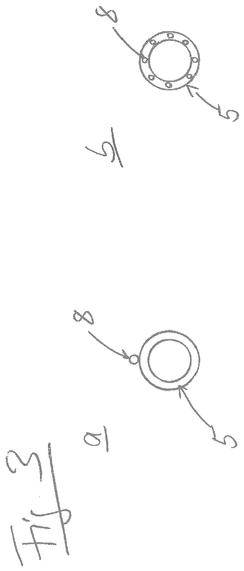

In Figur 3 sind zwei Ausführungsvarianten des Pumpenschlauchs 5, wie er bei den Vorrichtung gemäß Figuren 1, 2 Verwendung findet, in einem schematischen Querschnitt dargestellt.

Bei der Ausführungsvariante gemäß Figur 3a ist die Lichtleitfaser 8 auf der Außenseite des Pumpenschlauchs 5 angeordnet. Die Lichtleitfaser 8 kann dabei nachträglich auf den Pumpenschlauch 5 aufgeklebt sein. Es ist aber auch möglich, dass die Lichtleitfaser 8 mit dem Pumpenschlauch 5 coextrudiert ist.

Der Pumpenschlauch 5 gemäß Figur 3b ist als Multilumenschlauch ausgeführt, wobei die Lichtleitfaser 8 durch eine Kammer des Multilumenschlauches geführt ist. Der Pumpenschlauch 5 ist dabei zumindest an den Stellen, an denen Licht aus der Lichtleitfaser 8 austritt - also am zweiten Ende 10 und/oder den Störstellen 11 - transparent. Neben der zentralen Kammer für den Flüssigkeitstransport können durch die übrigen Kammern des Multilumenschlauchs bspw. Lichtleitfasern für Endoskopiebeleuchtung oder Laserapplikationen etc. geführt sein. Die Lichtleitfaser 8 kann mit dem Multilumenschlauch coextrudiert sein oder nachträglich in einen bereits vorhandenen Multilumenschlauch eingeführt werden.

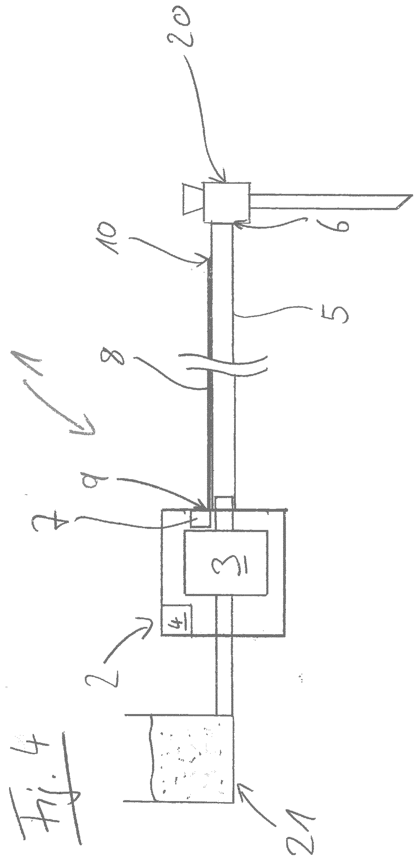

In Figur 4 ist eine erfindungsgemäße Anordnung aus einer Pumpenvorrichtung 1 und einem Endoskop 20 dargestellt. Die Pumpenvorrichtung 1 entspricht dabei derjenigen aus Figur 1, weshalb auf die dortigen Ausführungen verwiesen wird. Das Endoskop 20 weist neben seiner Endoskopoptik einen Zufuhrkanal für Spülflüssigkeit auf, mit der ein zu untersuchender Hohlraum gefüllt und gespült werden kann. Entsprechende Endoskope sind aus dem Stand der Technik bekannt.

Die Pumpenvorrichtung 1 ist bei einer entsprechenden Anordnung zur Zuführung von Spülflüssigkeit aus einem Reservoir 21 ausgebildet. Der Betriebszustand der Pumpeneinheit 2 der Pumpenvorrichtung 1 wird dem Operateur dabei durch den Lichtpunkt am zweiten Ende 10 der Lichtleitfaser 8 angezeigt, das aufgrund seiner Anordnung am Ende 6 des Pumpenschlauchs 5 nah am Endoskop 20 angeordnet ist (vgl. Ausführungen zu Figur 1). Der Operateur erhält somit Informationen über den Betriebszustand der Pumpeneinheit 2 in unmittelbarer Nähe zum Operationsgebiet, sodass er diese Informationen beiläufig und ohne Mühen wahrnehmen kann.

## Patentansprüche

1. Pumpenvorrichtung (1) zur Verwendung in Operationssälen, umfassend eine Pumpeneinheit (2) mit einer Pumpe (3) und einer Steuerungseinheit (4), und einen an die Pumpeneinheit (2) angeschlossenen Pumpenschlauch (5), wobei der Pumpenschlauch (7) eine Lichtleitfaser (8) aufweist, die sich entlang des Pumpenschlauchs (5) erstreckt, **dadurch gekennzeichnet, dass**
die Pumpeneinheit (2) eine durch die Steuerungseinheit (4) zur Anzeige des Betriebszustandes der Pumpeneinheit (2) ansteuerbare Lichtquelle (7) umfasst, wobei das erste Ende (9) der Lichtleitfaser (8) zur Einkopplung von Licht der ansteuerbaren Lichtquelle (7) angeordnet ist.

2. Vorrichtung nach Anspruch 1,
**dadurch gekennzeichnet, dass**
die Lichtquelle (7) zur Abgabe einer Strahlung außerhalb des sichtbaren Bereichs, vorzugsweise UV-Licht, ausgebildet ist und, vorzugsweise am zweiten Ende (10), ein Umwandlungselement zur Umwandlung der Strahlung der Lichtquelle (7) in sichtbares Licht vorgesehen ist.

3. Vorrichtung nach einem der vorhergehenden Ansprüche,
**dadurch gekennzeichnet, dass**
die Lichtleitfaser (8) zwischen ihrem ersten Ende (9) und ihrem zweiten Ende (10) eine oder mehrere Störstellen (11) zum Lichtaustritt aufweist, wobei die Störstellen (11) vorzugsweise Kerben auf der Außenseite der Lichtleitfaser (8) sind.

4. Vorrichtung nach Anspruch 3,
**dadurch gekennzeichnet, dass**
die Abstände zwischen zwei jeweils benachbarten Störstellen (11) ausgehend vom ersten Ende (8) der Lichtleitfaser (8) zum zweiten Ende (10) hin abnehmen.

5. Vorrichtung nach einem der vorhergehenden Ansprüche,
**dadurch gekennzeichnet, dass**
die Lichtquelle (7) farbveränderlich ist, wobei vorzugsweise die Steuerungseinheit (4) zur Steuerung der Farbveränderung der Lichtquelle (7) ausgebildet ist.

6. Vorrichtung nach Anspruch 5,
**dadurch gekennzeichnet, dass**
die Steuerungseinrichtung (4) dazu ausgebildet ist, die Farbe der Lichtquelle (7) in Abhängigkeit von der Durchflussrichtung zu verändern.

7. Vorrichtung nach einem der vorhergehenden Ansprüche,
**dadurch gekennzeichnet, dass**
die Steuerungseinrichtung (4) dazu ausgebildet ist, die Lichtquelle (7) in Abhängigkeit der Durchflussrate pulsieren oder blinken zu lassen.

8. Vorrichtung nach einem der vorhergehenden Ansprüche,
**dadurch gekennzeichnet, dass**
die Pumpeneinrichtung (2) einen oder mehrere Sensoren (15) zur Überwachung von Betriebsparametern der Pumpeneinheit (2) und/oder von Eigenschaften des zu pumpenden Fluids aufweist, wobei die Steuerungseinrichtung (4) vorzugsweise zur Anzeige der Messwerte des oder der Sensoren (15) durch die Lichtquelle (7) ausgebildet ist.

9. Vorrichtung nach einem der vorhergehenden Ansprüche,
**dadurch gekennzeichnet, dass**
die Lichtleitfaser (8) auf der Außenseite des Pumpenschlauchs (5) angeordnet ist, wobei die Lichtleitfaser (8) vorzugsweise auf den Pumpenschlauch (5) aufgeklebt oder mit diesem coextrudiert ist, oder der Pumpenschlauch (5) ein Multilumenschlauch ist, wobei die Lichtleitfaser (8) durch eine Kammer des Multilumenschlauchs geführt oder mit dem Multilumenschlauch coextrudiert ist.

10. Vorrichtung nach einem der vorhergehenden Ansprüche,
**dadurch gekennzeichnet, dass**
die ansteuerbare Lichtquelle (7) am Pumpenschlauch (5), vorzugsweise am freien Ende (6) des Pumpenschlauchs (5) angeordnet ist und über eine elektrische Leitung, die sich anstelle der Lichtleitfaser (8) entlang des Pumpenschlauchs (5) erstreckt, mit der Steuerungseinheit (4) der Pumpeneinheit (2) verbunden ist.

11. Anordnung umfassend eine Pumpenvorrichtung (1) und ein Endoskop (20),
**dadurch gekennzeichnet, dass**
die Pumpenvorrichtung (1) gemäß einem der Ansprüche 1 bis 10 und das ausgebildet Endoskop (20) zur Zuführung von Spülflüssigkeit in das Operationsgebiet ausgebildet ist, wobei das freie Ende (6) des Pumpenschlauchs (5) zur Zuführung von Spülflüssigkeit mit dem Endoskop (20) verbunden ist.

## Claims

1. Pump device (1) for use in operating theaters, comprising a pump unit (2) with a pump (3) and a control unit (4), and a pump tube (5) connected to the pump unit (2), wherein the pump tube (7) has an optical fiber (8) extending along the pump tube (5),
**characterized in that**
the pump unit (2) comprises a light source (7) actuatable by the control unit (4) to display the operating state of the pump unit (2), wherein the first end (9) of the optical fiber (8) is arranged for coupling-in light from the actuatable light source (7).

2. Device according to Claim 1,
**characterized in that**
the light source (7) is embodied to output radiation outside of the visible range, preferably UV light, and provision is made, preferably at the second end (10), of a conversion element for converting the radiation from the light source (7) into visible light.

3. Device according to one of the preceding claims,
**characterized in that**
the optical fiber (8) has one or more faults (11) for light exit between the first end (9) and the second end (10) thereof, the faults (11) preferably being notches on the outside of the optical fiber (8).

4. Device according to Claim 3,
**characterized in that**
the distances between the two faults (11) adjacent to one another in each case decrease toward the second end (10) proceeding from the first end (8) of the optical fiber (8) .

5. Device according to one of the preceding claims,
**characterized in that**
the light source (7) is variable in color, the control unit (4) preferably being embodied to control the color change of the light source (7).

6. Device according to Claim 5,
**characterized in that**
the control apparatus (4) is embodied to change the color of the light source (7) in a manner dependent on the flow direction.

7. Device according to one of the preceding claims,
**characterized in that**
the control apparatus (4) is embodied to let the light source (7) pulsate or blink depending on the flow rate.

8. Device according to one of the preceding claims,
**characterized in that**
the pump apparatus (2) has one or more sensors (15) for monitoring operational parameters of the pump unit (2) and/or properties of the fluid to be pumped, the control apparatus (4) preferably being embodied to display the measured values from the sensor or sensors (15) by way of the light source (7).

9. Device according to one of the preceding claims,
**characterized in that**
the optical fiber (8) is arranged on the outer side of the pump tube (5), the optical fiber (8) preferably being adhesively bonded onto the pump tube (5) or being coextruded with the latter, or the pump tube (5) being a multi-lumen tube, with the optical fiber (8) being guided through a chamber of the multi-lumen tube or being coextruded with the multi-lumen tube.

10. Device according to one of the preceding claims,
**characterized in that**
the actuatable light source (7) is arranged at the pump tube (5), preferably at the free end (6) of the pump tube (5), and connected to the control unit (4) of the pump unit (2) by means of an electric line, which extends along the pump tube (5) in place of the optical fiber (8).

11. Arrangement comprising a pump device (1) and an endoscope (20),
**characterized in that**
the pump device (1) according to one of Claims 1 to 10 and the embodied endoscope (20) are embodied to supply rinsing liquid into the operating region, wherein the free end (6) of the pump tube (5) is connected to the endoscope (20) for supplying rinsing liquid.

## Revendications

1. Dispositif à pompe (1) destiné à être utilisé dans des salles d'opération, ledit dispositif comprenant une unité de pompe (2), pourvue d'une pompe (3) et d'une unité de commande (4), et un tuyau de pompe (5) raccordé à l'unité de pompe (2), le tuyau de pompe (7) comportant une fibre optique (8) qui s'étend le long du tuyau de pompe (5),
**caractérisé en ce que**
l'unité de pompe (2) comprend une source de lumière (7) qui peut être commandée par l'unité de commande (4) pour afficher l'état de fonctionnement de l'unité de pompe (2),
la première extrémité (9) de la fibre optique (8) étant disposée de façon à injecter par couplage la lumière de la source lumineuse commandable (7).

2. Dispositif selon la revendication 1,
**caractérisé en ce que**
la source de lumière (7) est conçue pour délivrer un rayonnement en dehors du domaine visible, de préférence de la lumière UV, et un élément de conversion est prévu, de préférence à la deuxième extrémité (10), pour convertir le rayonnement de la source de lumière (7) en lumière visible.

3. Dispositif selon l'une des revendications précédentes,
**caractérisé en ce que**
la fibre optique (8) comporte entre sa première extrémité (9) et sa deuxième extrémité (10) au moins une irrégularité (11) de sortie de lumière, les irrégularités (11) étant de préférence des encoches situées du côté extérieur de la fibre optique (8).

4. Dispositif selon la revendication 3,
**caractérisé en ce que**
la distance entre deux irrégularités adjacentes (11) diminue de la première extrémité (8) de la fibre optique (8) à la deuxième extrémité (10).

5. Dispositif selon l'une des revendications précédentes,
**caractérisé en ce que**
la source de lumière (7) peut changer de couleur, l'unité de commande (4) étant de préférence conçue pour commander le changement de couleur de la source de lumière (7).

6. Dispositif selon la revendication 5,
**caractérisé en ce que**
l'unité de commande (4) est conçue pour changer la couleur de la source de lumière (7) en fonction du sens d'écoulement.

7. Dispositif selon l'une des revendications précédentes,
**caractérisé en ce que**
l'unité de commande (4) est conçue pour faire pulser ou clignoter la source de lumière (7) en fonction du débit.

8. Dispositif selon l'une des revendications précédentes,
**caractérisé en ce que**
l'unité de pompe (2) comporte au moins un capteur (15) destiné à surveiller les paramètres de fonctionnement de l'unité de pompe (2) et/ou les propriétés du fluide à pomper, l'unité de commande (4) étant conçu de préférence pour afficher les valeurs de mesure de l'au moins un capteur (15) par le biais de la source de lumière (7).

9. Dispositif selon l'une des revendications précédentes,
**caractérisé en ce que**
la fibre optique (8) est disposée du côté extérieur du tuyau de pompe (5), la fibre optique (8) étant collée ou co-extrudée de préférence sur le tuyau de pompe (5), ou le tuyau de pompe (5) est un tuyau à plusieurs lumières, la fibre optique (8) étant guidée à travers une chambre du tuyau à plusieurs lumières ou co-extrudée avec le tuyau à plusieurs lumières.

10. Dispositif selon l'une des revendications précédentes,
**caractérisé en ce que**
la source de lumière commandable (7) est disposée sur le tuyau de pompe (5), de préférence à l'extrémité libre (6) du tuyau de pompe (5) et est reliée à l'unité de commande (4) de l'unité de pompe (2) par le biais d'une ligne électrique qui s'étend le long du tuyau de pompe (5) à la place de la fibre optique (8).

11. Ensemble comprenant un dispositif à pompe (1) et un endoscope (20),
**caractérisé en ce que**
le dispositif à pompe (1) selon l'une des revendications 1 à 10 et l'endoscope (20) sont conçus pour amener du liquide de lavage dans la zone d'opération, l'extrémité libre (6) du tuyau de pompe (5) étant reliée à l'endoscope (20) pour amener du fluide de lavage.
